# EUROPÄISCHE PATENTSCHRIFT

(11) **EP 3 235 454 B1**
(45) Veröffentlichungstag und Bekanntmachung des Hinweises auf die Patenterteilung: **25.12.2019**
(21) Anmeldenummer: 16000867.8
(22) Anmeldetag: 18.04.2016
(51) Int. Cl.: A61B 17/22, A61B 17/225, A61N 7/00

(54) **VORRICHTUNG UND SYSTEM ZUR ABGABE VON STOSSWELLEN**
DEVICE AND SYSTEM FOR DISPENSING SHOCK WAVES
DISPOSITIF ET SYSTEME DE DISTRIBUTION D'ONDES DE CHOC

(43) Veröffentlichungstag der Anmeldung: 25.10.2017
(73) Patentinhaber: MTS Medical UG, 78467 Konstanz (DE)
(72) Erfinder: Reitmajer, Ralph, D-78467 Konstanz (DE)

(56) Entgegenhaltungen:
- CN-U- 201 532 587
- DE-A1- 10 354 147
- US-A- 5 560 362
- US-A- 6 036 661
- US-A1- 2004 171 970
- US-A1- 2007 016 112
- US-A1- 2008 161 743
- US-B1- 6 533 738

## Beschreibung

Die Erfindung betrifft eine Vorrichtung zur Abgabe von Stosswellen, ein System zur Erzeugung und Abgabe von Stosswellen, ein Verfahren zum Austauschen eines Behandlungskopfes, ein Verfahren zum Herstellen eines Systems und einen Grundkörper für eine Steckereinheit gemäss den Oberbegriffen der unabhängigen Ansprüche.

Stosswellen-Vorrichtungen werden in der Medizintechnik in verschiedenen Bereichen eingesetzt, beispielsweis in der Urologie als Nierensteinzertrümmerer, in der Orthopädie bei nicht heilenden Knochenbrüchen oder bei Sehenansatztendinosen. Weitere Anwendungsgebiete sind beispielsweise in der DE 10 2008 049 924 genannt. In jüngerer Zeit werden Stosswellen auch in der Dermatologie zur Heilung von chronischen Wunden und in der Urologie zur Behandlung von erektiler Dysfunktion eingesetzt.

Stosswellen können von elektrohydraulischen, elektromagnetischen oder piezoelektrischen Systemen erzeugt werden. Die Stosswellen müssen nach ihrer Erzeugung in den zu therapierenden Körper eingekoppelt werden. Weil das zu durchdringende Weichgewebe des Körpers vergleichbare akustische Eigenschaften wie Wasser besitzt, werden die Stosswellen bevorzugt in Wasser erzeugt oder zunächst an Wasser abgegeben. Der Behandlungskopf des Stosswellenerzeugungssystems enthält daher in der Regel ein Flüssigkeitsreservoir für Wasser. Das Wasser kann gleichzeitig zur Ableitung der entstehenden Prozesswärme, zum Abführen von Gasen und/oder zur Spülung des Elektodenabbrandes während der Erzeugung der Stosswelle dienen.

Ferner kann über eine Koppelmembran und unterschiedliche Wasserdrücke in der Membran die Ankopplung an unterschiedliche Körperkonturen und -dicken erreicht werden, um das gewünschte Zielgebiet mit den Stosswellen zu erreichen.

In dem verwendeten Wasser sind jedoch auf natürliche Weise Gase, die in der Atmosphäre vorkommen, nämlich O2, N2 und CO2 gelöst. Zusätzlich entstehen bei der elektrohydraulischen Stosswellenerzeugung während des Funkensprungs durch Stossionisation Sauerstoff und Wasserstoff. Selbst wenn der Wasserstoff durch Diffusion aus dem System entweicht, bleibt der Sauerstoff gelöst oder gasförmig im System zurück.

Die Gase beeinträchtigen jedoch die Stosswellenausbreitung. Sie können im Zugwellenanteil der Stosswellen zu Kavitationsblasen führen. Die im Schallfeld befindlichen Gasblasen behindern durch Streuung und Reflektion die Stosswellenausbreitung. Als Folge kann eine Reduktion des Gerätewirkungsgrades entstehen.

Zur Vermeidung dieses Verlustes werden in der Regel spezielle Entgasungsvorrichtungen in den Wasserkreislauf eingebaut.

Die EP2023829 schlägt vor, das Wasser chemisch zu entgasen, alternativ kann vorgesehen sein, das Wasser zu erhitzen, eine Unterdruckatmosphäre anzulegen, das Wassers in die Unterdruckatmosphäre zu versprühen oder eine semipermeable Membran einzusetzen.

Für viele Anwendungen ist es wichtig, dass der Behandlungskopf, von welchem die Stosswellen an den zu therapierenden Körper abgegeben werden, unabhängig von einer Basisstation, in welcher die notwendige Spannung zur Verfügung gestellt wird, bewegbar ist. Zudem kann es notwendig sein, dass der Behandlungskopf von der Basisstation abkoppelbar ist, zum Beispiel zu Wartungszwecken oder wenn für eine andere Anwendung ein Behandlungskopf mit einer anderen Abstrahlcharakteristik verwendet werden soll.

Typischerweise ist daher ein Wasserkreislauf vorgesehen, der zum einen das Wasserreservoir im Behandlungskopf und zum anderen eine Umwälzeinrichtung einschliesst. Die Umwälzeinrichtung umfasst mindestens einen Vorratstank, mindestens eine Pumpe und mindestens eine Entgasungsöffnung, bevorzugt ein Entgasungsventil. Die Umwälzeinrichtung ist in der Regel in der Basisstation angeordnet. Zum Abkoppeln des Behandlungskopfes muss daher in dem Wasserkreislauf eine Fluidkupplung vorgesehen sein.

In elektrohydraulische Systeme wird zwischen zwei in flüssigem Medium befindlichen Elektrodenspitzen eine Spannung angelegt, bei deren Durchbruch, also bei deren schlagartiger Entladung, das flüssige Medium explosionsartig verdampft und sich ein Plasma bildet. Von diesem Plasma löst sich eine Druckwelle ab, die in der Regel über einen Reflektor, zum Beispiel in Form eines Ellipsoids oder Paraboloids, reflektiert wird. Bei der Entladung nutzen die Elektrodenspitzen ab. Aus der DE 20 2007 012 283 ist bekannt, dass es für die Lebensdauer der Elektrodeneinheiten vorteilhaft ist, wenn nicht lösliches Material, typischerweise in Form von Graphit- oder Aktivkohleteilchen, zwischen den Elektroden angeordnet ist.

Diese Teilchen, die sich im Wasserkreislauf befinden, wirken sich störend auf die Dichtheit einer Fluidkupplung aus.

Aus der DE 197 18 513 ist eine Vorrichtung bekannt, in welcher der Reflektor und die Elektroden zusammen mit dem Flüssigkeitsvolumen, das von dem Reflektor und einer Koppelmembran eingeschlossen ist, eine auswechselbare Einheit bilden. Die Vorrichtung verzichtet also auf eine Umwälzeinrichtung. Die Entgasung, bzw., der Austausch der Flüssigkeit, findet bei einem Austausch der Elektroden statt. Dies ist nur sinnvoll, wenn die maximal verträgliche Gasmenge nicht erreicht ist, bevor die Elektroden verbraucht sind. Für langlebige Elektrodensysteme kommt diese Lösung daher nicht in Frage.

US 6 533 738 B1 offenbart eine Vorrichtung zur Abgabe von Stosswellen, mit einem Behandlungskopf, in welchem eine Stosswellenerzeugungsvorrichtung angeordnet ist und der ein Flüssigkeitsreservoir umfasst.US 2004/171970 A1 offenbart eine Ultraschallbehandlungsvorrichtung mit einer Pumpe für Kühlflüssigkeit im Behandlungskopf.US 2007/016112 A1 offenbart eine Vorrichtung zur Abgabe von Stosswellen, mit einem Behandlungskopf, in welchem eine Stosswellenerzeugungsvorrichtung angeordnet ist und der ein Flüssigkeitsreservoir umfasst, wobei die Stosswellenerzeugungsvorrichtung eine elektrohydraulische Stosswellenerzeugungsvorrichtung ist und Elektroden in einem Reflektor angeordnet sind.

Es besteht daher die Aufgabe der vorliegenden Erfindung, eine Vorrichtung zur Abgabe von Stosswellen, ein Verfahren zum Austauschen eines Behandlungskopfes und ein Verfahren zum Herstellen einer Vorrichtung zur Abgabe von Stosswellen zur Verfügung zu stellen, welche die Nachteile des Bekannten vermeiden, welche einen langlebigen Betrieb ermöglichen und eine bequeme Handhabung erlauben.

Die Aufgabe wird gelöst durch eine Vorrichtung zur Abgabe von Stosswellen gemäß Anspruch 1. Die Vorrichtung weist einen Behandlungskopf auf, in welchem eine Stosswellenerzeugungsvorrichtung angeordnet ist und der ein Flüssigkeitsreservoir Bevorzugte Ausführungsformen werden in den Unteransprüche definiert.

Das Flüssigkeitsreservoir kann in Abgaberichtung der Stosswellen mit einer Koppelmembran abgeschlossen sein. Das Flüssigkeitsreservoir ist mit Flüssigkeit befüllbar, sodass für den Betrieb eine gefülltes Flüssigkeitsreservoir zur Verfügung steht.

Die Vorrichtung weist ausserdem eine Steckereinheit auf, die Steckkontakte zum elektrischen Kontaktieren der Stosswellenerzeugungsvorrichtung mit einer Basisstation umfasst.

Die elektrischen Kontakte dienen einerseits dazu, die notwendige elektrische Energie für die Stosswellenerzeugungsvorrichtung bereit zu stellen, wozu zum Beispiel Hochspannungssteckkontakte notwendig sein können, sie können andererseits Signale zur Steuerung der Stosswellenerzeugungsvorrichtung übermitteln.

Die Vorrichtung weist zudem eine Umwälzeinrichtung auf, die mindestens einen Vorratstank, mindestens eine Pumpe und mindestens eine Entgasungsöffnung, insbesondere ein Entgasungsventil, umfasst.

Der Vorratstank steht in einer Fluidverbindung mit dem Flüssigkeitsreservoir, so dass die Umwälzeinrichtung und das Flüssigkeitsreservoir Bestandteile eines abschliessbaren Flüssigkeitskreislaufs sind, in dem bevorzugt Wasser zirkulieren kann.

Die Pumpe sorgt dafür, dass die Flüssigkeit in dem Flüssigkeitskreislauf umgewälzt wird und dafür, dass im Flüsskeitsreservoir ein genügend grosser Flüssigkeitsdruck vorhanden ist.

Die Entgasungsöffnung lässt in der Flüssigkeit entstehendes Gas entweichen.

Die Vorrichtung weist insbesondere eine Verbindungsleitung zwischen dem Behandlungskopf und der Steckereinheit auf. In der Verbindungsleitung verlaufen zumindest elektrische Leitungen zwischen Steckkontakten der Steckereinheit und der Stosswellenerzeugungsvorrichtung.

Die Verbindungsleitung überbrückt den Abstand zwischen der Basisstation und dem Behandlungskopf. Eine genügend lange Verbindungsleitung erlaubt die Behandlung in schwieriger erreichbaren Therapiebereichen, die nicht ohne weiteres an die Basisstation herangeführt werden können, und eine Behandlung, bei welcher der Behandlungskopf während der Behandlung im Therapiebereich bewegt wird.

Erfindungsgemäss ist die Umwälzeinrichtung baulich in den Behandlungskopf und/oder die Steckereinheit integriert. Insbesondere ist die Umwälzeinrichtung baulich in den Behandlungskopf, die Steckereinheit und/oder die Verbindungleitung integriert.

Dabei können alle Komponenten der Umwälzeinrichtung in der Steckereinheit, dem Behandlungskopf oder in der Verbindungsleitung angeordnet sein, oder nur jeweils ein Teil der Komponenten.

Bevorzugt ist die Umwälzeinrichtung, also der Vorratstank, die Pumpe und das Entgasungsventil, in die Steckereinheit integriert.

Da die Steckereinheit während des Betriebes mit der Basisstation verbunden ist, besteht gegenüber einer herkömmlichen Vorrichtung, bei der die Umwälzeinrichtung und das damit verbundene Gewicht in der Basisstation angeordnet ist, kein Nachteil. Die Umwälzeinrichtung wird während der Behandlung nicht bewegt und muss auch nicht gehoben werden.

Zudem kann die Umwälzeinrichtung während des Betriebes ortsfest verbleiben, was vorteilhaft für den Betrieb der Pumpe ist.

Die Umwälzeinrichtung ist so angeordnet, dass die erfindungsgemässe Vorrichtung zur Abgabe von Stosswellen insbesondere fluidmässig abgeschlossen gegenüber der Basisstation ist. Alle Bestandteile des Flüssigkeitskreislaufs befinden sich in der erfindungsgemässen Vorrichtung, sodass keine Fluidkupplung gegenüber der Basisstation oder einer andererweitigen externen Flüssigkeitsversorgung benötigt wird.

Bei der Stosswellenerzeugungsvorrichtung kann es sich um eine elektrohydraulische, elektromagnetische oder piezoelektrische Stosswellenerzeugungsvorrichtung handeln.

Gemäß der Erfindung, ist die Stosswellenerzeugungsvorrichtung eine elektrohydraulische Stosswellenerzeugungsvorrichtung, wobei Elektroden in einem Reflektor angeordnet sind. Typischerweise sind zwei Elektroden so angeordnet, dass sich der Spalt zwischen den Elektrodenspitzen im Fokus des Reflektors befindet. Die Elektroden sind insbesondere nachregelbar, sodass der Spalt stets die optimale Position im Reflektor einnehmen kann, der Abstand zwischen den Elektroden auf die gewünschte Länge einstellbar ist und/oder dem Abbrand der Elektroden durch Nachführung begegnet werden kann.

Insbesondere ist der Reflektor mit einer Flüssigkeit befüllbar, wobei der Reflektor in Richtung der Stosswellenabgabe von einer Koppelmembran abgeschlossen wird. Der Reflektor bildet somit ein mit einer Koppelmembran im Wesentlichen abgeschlossenes Flüssigkeitsreservoir.

In einer vorteilhaften Ausführung der erfindungsgemässen Vorrichtung ist das Flüssigkeitsreservoir im Behandlungskopf mit einer Flüssigkeit befüllt, die nicht lösliches Material enthält. Bevorzugt enthält die Flüssigkeit leitende, halbleitenden, polarisierbare Teilchen, Aktivkohleteilchen und/oder Graphitteilchen. Bei der Flüssigkeit handelt es sich bevorzugt um Wasser.

Die Teilchen begünstigen den Betrieb insbesondere eines elektrohydraulischen Stosswellensystems. Die Teilchen begünstigen den elektrischen Durchschlag, verkürzen den effektiven Abstand zwischen den Elektroden und tragen zu einer längeren Lebensdauer, also zu einer erhöhten Anzahl von erzeugbaren Stosswellen mit einem Elektrodenpaar, bei.

Die Teilchen werden zusammen mit der Flüssigkeit in dem Flüssigkeitskreislauf zirkuliert. Eine gleichmässige Verteilung der Teilchen in der Flüssigkeit stellt sich zum einen durch die Pumpe ein, zum anderen durch die Stosswelle.

Vorteilhafterweise ist die mindestens eine Entgasungsöffnung, insbesondere das Entgasungsventil, insbesondere für den Transport, verschliessbar. Das Entgasungsventil kann auch zwischen den oder während der Behandlungen verschlossen werden, damit die Flüssigkeit nicht verdampft.

Das Entgasungsventil umfasst beispielsweise Öffnungen, die so angeordnet sind, dass zwar Gas entweichen kann, die Flüssigkeit aufgrund der Schwerkraft aber im System beleibt. Wird die Vorrichtung von der Basisstation gelöst oder transportiert, so ist die Ausrichtung der Öffnungen nicht mehr sichergestellt und Flüssigkeit kann aus dem unverschlossenen Entgasungsventil tropfen. Die Öffnungen können beispielweise mit einem Schieberelement verschlossen werden.

Es kann ein mechanisches oder elektromagnetisch betriebenes Ventil vorgesehen sein, das die Entgasungsöffnungen automatisch schliesst, wenn die Vorrichtung von der Basisstation entfernt wird und öffnet, sobald die Vorrichtung an die Basisstation angekoppelt wird.

Es kann auch eine Kombination eine mechanischen Ventils und eines elektromagnetischen Ventils vorgesehen werden. Beispielweise kann ein elektromagnetisches Ventil mit kleinem Durchfluss-Durchmesser vorgesehen sein, welches bei Nicht-Benutzung der Vorrichtung geschlossen wird und welches verhindert, dass im Ruhezustand Flüssigkeit verdunstet.

Zusätzlich kann ein mechanisches Ventil schliessen, wenn die Vorrichtung von der Basisstation entfernt wird und zu erwarten ist, dass die Ausrichtung der Vorrichtung nicht beibehalten wird. Wird die Basisstation ausgeschaltet und der Applikator verbleibt angesteckt, kann das mechanische Ventil geöffnet bleiben.

Das mechanische Ventil kann einen vorspannbaren, beweglichen Stössel mit einer Dichtung, zum Beispiel einem O-Ring, umfassen, der anschlägt, wenn die Vorrichtung von der Basisstation getrennt wird. Bevorzugt ist an der Basisstation ein Gegenlager für den Stössel angebracht, der das mechanische Ventil beim Verbinden der Vorrichtung mit der Basisstation in die geöffnete Position drückt.

Elektromagnetisches Ventil und mechanisches Ventil sind bevorzugt in Reihe geschaltet. Das mechanische Ventil verhindert ein Verstopfen des elektromagnetischen Ventils während des Transports der Vorrichtung.
In einer vorteilhaften Ausführung der Erfindung umfasst die Vorrichtung eine Verbindungsleitung zwischen Behandlungskopf und Steckereinheit, in welcher Fluidleitungen angeordnet sind. Diese können eine Fluidverbindung von dem Vorratsbehälter zur dem Flüssigkeitsreservoir im Behandlungskopf herstellen.

Bevorzugt umfasst die Steckereinheit ein Gehäuse, insbesondere ein Kunststoffgehäuse, in welchem ein Vorratstank, Flüssigkeitsleitungen und/oder eine Aufnahme für eine Pumpe integriert sind.

Das Gehäuse kann so gefertigt sein, beispielsweis im Spritzgussverfahren oder im 3D-Druckverfahren, dass der Vorratstank, die Flüssigkeitsleitungen und die Pumpenaufnahme fluiddicht in dem Gehäuse ausgebildet sind.

Bevorzugt ist an dem Vorratstank oder an den Fluidleitungen mindestens eine Entgasungsöffnung, insbesondere ein Entgasungsventil, vorgesehen.

Insbesondere ist das Gehäuse für die Aufnahme elektrischer Steckkontakte und elektrischer Leitungen ausgelegt.

In einer vorteilhaften Ausführung umfasst die Steckereinheit elektrische Anschlussmittel und/oder elektronische Steuermittel zur Bedienung der Umwälzeinrichtung, insbesondere der Pumpe.

Bevorzugt besitzt die Steckereinheit einen 12 oder 24 V (Gleichspannung/Masse) Anschluss für den Betrieb der Pumpe und insbesondere zwei Steuerleitungen zur Drehzahlregulierung.

Die Steuermittel können ein Halbleiterbauelement umfassen und/oder eine elektrische Sicherung.
Der Vorratstank fasst bevorzugt ein Flüssigkeitvolumen zwischen 30 ml und 250 und/oder die Vorrichtung schliesst ein Flüssigkeitsvolumen von 80 ml bis 350 ml (gesamte Wassermenge) ein. Für Anwendungen, bei denen ein grosser Behandlungskopf verwendet wird, zum Beispiel in der Lithotripsie, muss ein entsprechend grösseres Volumen zugrunde gelegt werden.
Die Pumpe ist bevorzugt so ausgelegt, dass sie eine Leistung zwischen 1.5 bis 10 Watt verrichtet und/oder einen Durchsatz von 0.5 bis 3.0 Liter pro Minute erbringt und/oder einen Druck von 150 bis 1000 mbar erzeugt, sodass die Flüssigkeit auf eine Höhe zwischen 0.25m und 1m über dem Vorratstank gebracht werden kann.

Für Anwendungen, bei denen ein grosser Behandlungskopf verwendet wird, z.B. in der Lithotripsie, muss eine entsprechend grössere Leistung zugrunde gelegt werden.

Es kann ausserdem vorgesehen sein, den Behandlungskopf mit einer verformbaren Membran auszustatten und die Eindringtiefe der Behandlungskopfes mit dem Füllgrad des Flüssigkeitsreservoirs einzustellen. Je mehr Flüssigkeit in das Flüssigkeitsreservoir gebracht wird, desto mehr stülpt sich die Membran aus und desto kleiner ist die Eindringtiefe. Der Vorratstank und die Pumpe müssen in diesem Fall dafür ausgelegt sein, unterschiedliche Mengen von Flüssigkeit im Behandlungskopf zur Verfügung zu stellen.

Behandlungsköpfe mit einer festen Eindringtiefe haben zumeist eine festere Membran aus einem Kunststoff mit einer höheren Shore-Härte.

Der Flüssigkeitskreislauf, insbesondere die Umwälzeinrichtung kann ein Füllventil aufweisen, über welches Flüssigkeit in den Flüssigkeitskreislauf nachgefüllt werden kann. Alternativ kann die Nachfüllung des Flüssigkeitskreislaufs auch über die Entgasungsöffnung erfolgen, welche während des Betriebs geöffnet ist. Alternativ kann die Nachfüllung des Flüssigkeitskreislaufs auch über einen zweiten Vorratstank, welcher sich in der Basisstation befindet, erfolgen. Dabei sind in der Steckereinheit und der Basisstation noch Fluidkupplungen integriert, über welche Flüssigkeit durch eine Flüssigkeitspumpe vom zweiten Vorratstank der Basisstation in den Flüssigkeitskreislaufs der Vorrichtung nachgefüllt werden kann.

Der Behandlungskopf, die Steckereinheit, die Umwälzeinrichtung und insbesondere die Verbindungsleitung bilden gegenüber einer Basisstation eine auswechselbare Einheit. Für einen Wechsel des Behandlunsgkopfes ist es lediglich nötig, die elektrischen Anschlüsse zwischen Steckereinheit und Basisstation zu trennen, bzw. zu verbinden.

In einer vorteilhaften Weiterbildung der erfindungsgemässen Vorrichtung sind die zur Flüssigkeit weisenden Oberflächen des Flüssigkeitsreservoirs, des Vorratstanks, der Pumpe und/oder von in der Vorrichtung vorhandenen Flüssigkeitsleitungen mit einer Anti-Haft-Beschichtung versehen, damit Aktivkohle- oder GraphitTeilchen sich nicht ablagern können. Als Anti-Haft-Beschichtung können flüssige Anti-Haft-Mittel verwendet werden, mit denen die Oberflächen ausgekleidet werden. Es kann auch eine Plasmabeschichtung vorgesehen sein. Die Anti-Haft-Beschichtung kann Nanopartikel umfassen, die zu einem Lotusblüteneffekt führen.

Die der Erfindung zugrundeliegende Aufgabe wird ausserdem gelöst durch ein System gemäß Anspruch 12, zur Erzeugung und Abgabe von Stosswellen mit mindestens einer Vorrichtung wie oben beschrieben und einer elektrische Basisstation, die eine Steckeraufnahme für die Steckereinheit aufweist.

Die Basisstation weist insbesondere einen Hochspannungskondensator auf.

Ein solches System kann dem Benutzer mit mehreren Vorrichtungen zur Abgabe von Stosswellen zur Verfügung gestellt werden. Dabei können Behandlungsköpfe mit unterschiedlichen Abstrahlcharakteristiken für verschiedene Anwendungsgebiete vorgesehene sein.

Alternativ oder zusätzlich können Ersatzvorrichtungen vorhanden sein, die zum Beispiel zum Einsatz kommen, wenn Elektroden abgenutzt sind.

Die der Erfindung zugrundeliegende Aufgabe wird ausserdem gelöst durch ein Verfahren gemäß Anspruch 13, zum Austauschen eines Behandlungskopfes in einem System wie oben beschrieben, wobei der Behandlungskopf, die Steckereinheit, die Umwälzeinrichtung und insbesondere die Verbindungsleitung als bauliche Einheit von einer Basisstation entfernt und/oder an eine Basisstation montiert werden.

Die der Erfindung zugrundeliegende Aufgabe wird ausserdem gelöst durch ein Verfahren gemäß Anspruch 14, zum Herstellen einer Vorrichtung zur Abgabe von Stosswellen wie oben beschrieben. Ein Grundkörper für eine Steckereinheit wird im 3D-Druck Verfahren hergestellt. Der Grundkörper umfasst eine Aufnahme für elektrische Steckkontakte. Ausserdem kann der Grundkörper einen Vorratstank, eine Aufnahme für eine Pumpe, eine Aufnahme elektrische Leitungen und/oder Flüssigkeitsleitungen umfassen.

Die der Erfindung zugrundeliegende Aufgabe wird ausserdem gelöst durch einen Grundkörper gemäß Anspruch 15, eine Steckereinheit für eine Vorrichtung zur Abgabe von Stosswellen mit einem Behandlungskopf, in welchem eine Stosswellenerzeugungsvorrichtung angeordnet ist, insbesondere wie oben beschrieben.

Der Grundkörper umfasst eine Aufnahme für Steckkontakte zum elektrischen Kontaktieren der Stosswellenerzeugungsvorrichtung mit einer Basisstation. In dem Grundkörper sind ein Vorratstank und insbesondere eine Aufnahme für eine Pumpe (10) integriert.

Der Grundkörper ist aus Kunststoff, im 3D-Druckverfahren hergestellt

Im Folgenden wird die Erfindung anhand von in Figuren dargestellten Ausführungsbeispielen erläutert.

Hierbei zeigen
- Fig. 1: eine schematische Darstellung einer erfindungsgemässen Vorrichtung in perspektivischer Ansicht;
- Fig. 2: eine schematische Darstellung eines Gehäuses einer Steckereinheit in Draufsicht;
- Fig. 3: eine schematische Darstellung einer Steckereinheit in perspektivischer Ansicht;
- Fig. 4: eine schematische Darstellung einer Steckereinheit und einer Basisstation in perspektivischer Ansicht;
- Fig. 5a: eine schematische Darstellung eines ersten Beispiels für ein mechanisches Ventil in geschlossener Position;
- Fig. 5b: eine schematische Darstellung des ersten Beispiels für ein mechanisches Ventil in geöffneter Position;
- Fig. 6a: eine schematische Darstellung eines zweiten Beispiels für ein mechanisches Ventil in geschlossener Position;
- Fig. 6b: eine Detailansicht aus Figur 6a.

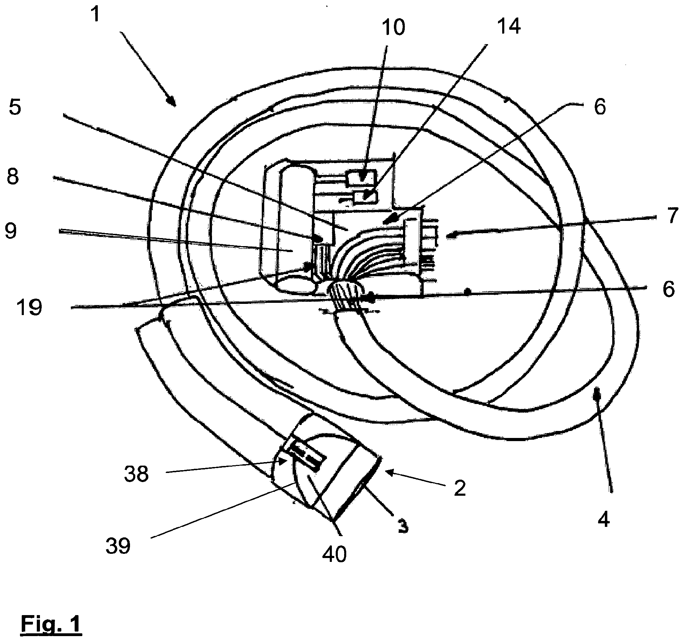

Figur 1 zeigt eine erfindungsgemässe Vorrichtung 1 zur Abgabe von Stosswellen. Die Vorrichtung 1 umfasst einen Behandlungskopf 2, in welchem eine nicht näher gezeigte Stosswellenerzeugungsvorrichtung angeordnet ist. Der Behandlungskopf 2 umfasst ein ebenfalls nicht näher gezeigtes Flüssigkeitsreservoir. Im vorliegenden Fall handelt es sich beispielhaft um eine elektrohydraulische Stosswellenerzeugungsvorrichtung, bei welcher zwei Elektroden 38 in einem Reflektor 39 angeordnet sind. Dieses bildet ein Flüssigkeitsreservoir 40, das mit einer Koppelmembran 3 abgeschlossen ist.
Der Behandlungskopf 2 ist mit einer Verbindungsleitung 4, in welcher elektrische Leitungen 6 verlaufen, an eine Steckereinheit 5 angeschlossen. In der Verbindungsleitung 4 sind ausserdem Fluidleitungen 19 angeordnet, welche eine Umwälzeinrichtung 8 mit dem Flüssigkeitsreservoir im Behandlungskopf 2 verbinden.

Die Steckereinheit 5 umfasst Steckkontakte 7 zum elektrischen Kontaktieren der Stosswellenerzeugungsvorrichtung mit einer Basisstation.

In die Steckereinheit 5 ist eine Umwälzeinrichtung 8 integriert, die einen Vorratstank 9, eine Pumpe 10 und ein Entgasungsventil 11 umfasst.

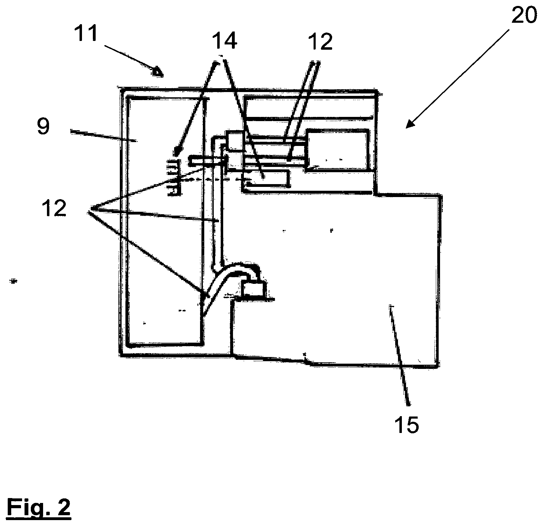

Die Steckereinheit 5 weist ein Gehäuse 11 auf, das in Figur 2 in einer schematische Ansicht gezeigt ist. Das Gehäuse 11 bildet einen Grundkörper 20 aus, der aus Kunststoff im 3D-Druck gefertigt ist. In das Gehäuse 11 sind ein Vorratstank 9, Flüssigkeitsleitungen 12 eine Aufnahme 13 für eine Pumpe integriert.

Der Vorratstank 9 ist mit Entgasungsöffnungen 14 ausgestattet.

Das Gehäuse 11 besitzt ausserdem eine Aufnahme 15 für elektrische Verbindungsmittel, also elektrische Leitungen 6 und Stecckontakte 7, auf.

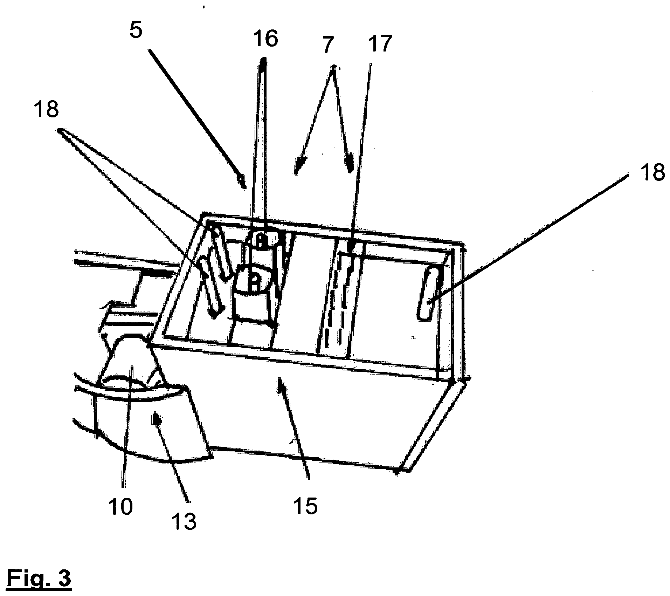

Figur 3 zeigt eine schematische Darstellung einer Steckereinheit 5 in perspektivischer Ansicht.

Die Steckereinheit 5 umfasst Steckkontakte 7, darunter Koaxialanschlüsse 16, mit denen die Hochspannung an den Elektroden zur Verfügung gestellt wird.

Die Steckkontakte 7 umfassen ausserdem Steuerkontakte 17 zu Steuerung der Stosswellenerzeugungsvorrichtung und der Pumpe.

An der Steckereinheit 5 sind ausserdem Führungsstecker 18 angeordnet, die dafür sorgen, dass bei einer Verbindung der Steckereinheit 5 mit einer Basisstation die Steckkontakte 7 zentriert in eine entsprechende Steckeraufnahme geführt werden, ohne dass Querkräfte auf die Steckkontakte 7 wirken.

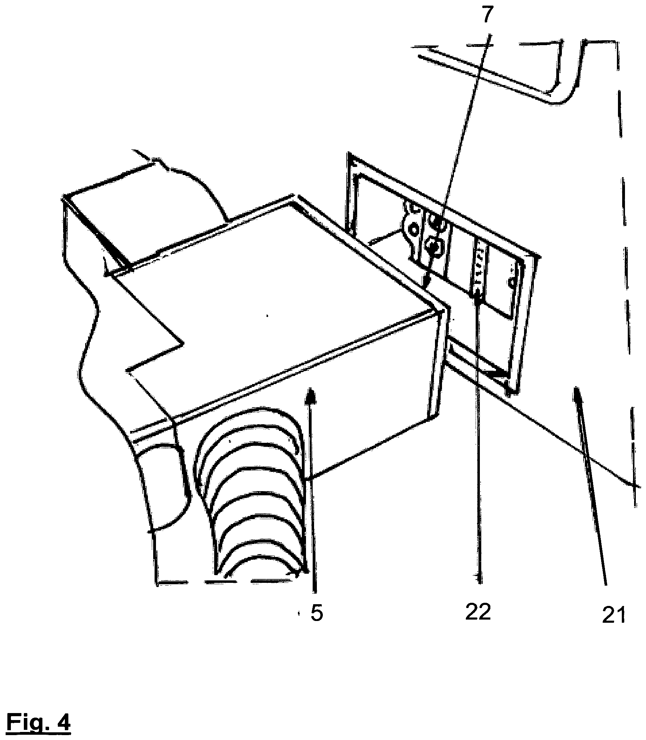

Figur 4 zeigt eine schematische Darstellung einer Steckereinheit 5 und einer Basisstation 21 in perspektivischer Ansicht.

Die Basisstation 21 besitzt eine Steckeraufnahme 22 für die Steckkontakte 7 und die Führungsstecker 18 der Steckereinheit 5.

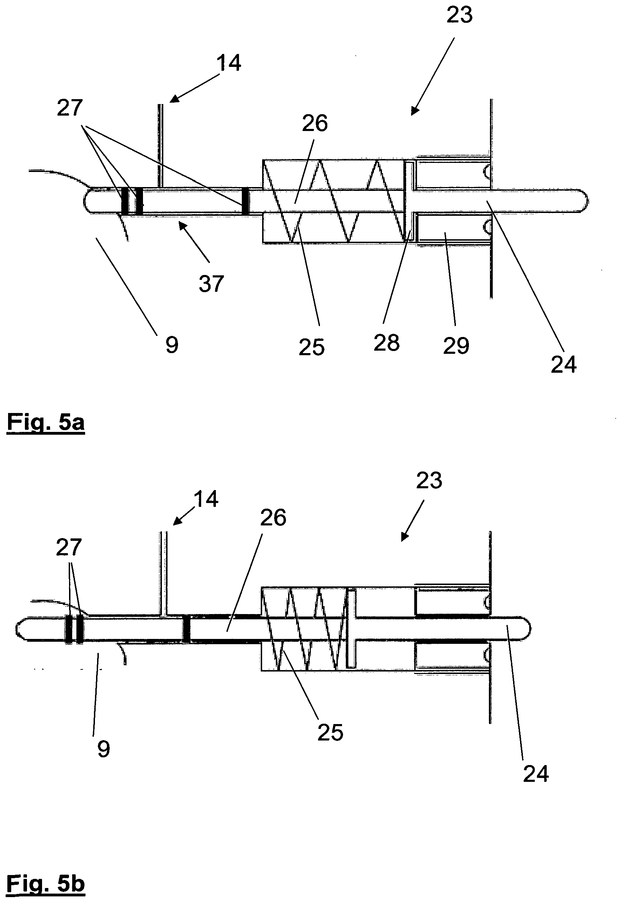

Figur 5a zeigt eine schematische Darstellung eines ersten Beispiels für ein mechanisches Ventil 23 zum Verschliessen einer Entgasungsöffnung 14 in geschlossener Position, Figur 5b das gleiche mechanische Ventil 23 in geöffneter Position.

Das Ventil 23 umfasst einen Stössel 24, der über eine Feder 25 vorgespannt ist.

An dem Stössel 24 ist in axialer Verlängerung eine Dichtstange 26 angebracht, auf welcher O-Ringe 27 angeordnet sind. Die Dichtstange 26 ist in einem Entgasungskanal 37 geführt, der einerseits in Fluidverbindung mit dem Vorratstank 9 und andererseits mit den Entgasungsöffnungen 14 steht.

Wenn die Vorrichtung 1 nicht mit der Basisstation 21 verbunden ist, drückt die Feder 25 den Kopf 28 des Stössels 24 gegen einen Anschlagblock 29. Die O-Ringe 27 sind dann in einer Position in dem Entgasungskanal 37, so dass die Fluidverbindung zwischen dem Vorratstank 9 und der Entgasungsöffnung 14 unterbrochen ist. Weder Gas noch Flüssigkeit können entweichen.

Ist die Vorrichtung 1 mit der Basisstation 21 verbunden, so wird der Stössel 24 und damit die Dichtstange 26 in Richtung des Vorratstanks 9 gedrückt. Die O-Ringe 27 nehmen dann eine Position ein, welcher den Austritt von Gas aus Vorratstank 9 und durch die Entgasungsöffnung 14 erlaubt.

In analoger Weise kann auch eine Vielzahl von Entgasungsöffnungen verschlossen werden, z.B. durch mehrere Entgasungsöffnungen an einer Entgasungskanal und/oder eine parallele Anordnung von Entgasungskanälen und Dichtstangen, die über einen gemeinsamen Stössel bewegbar sein können.

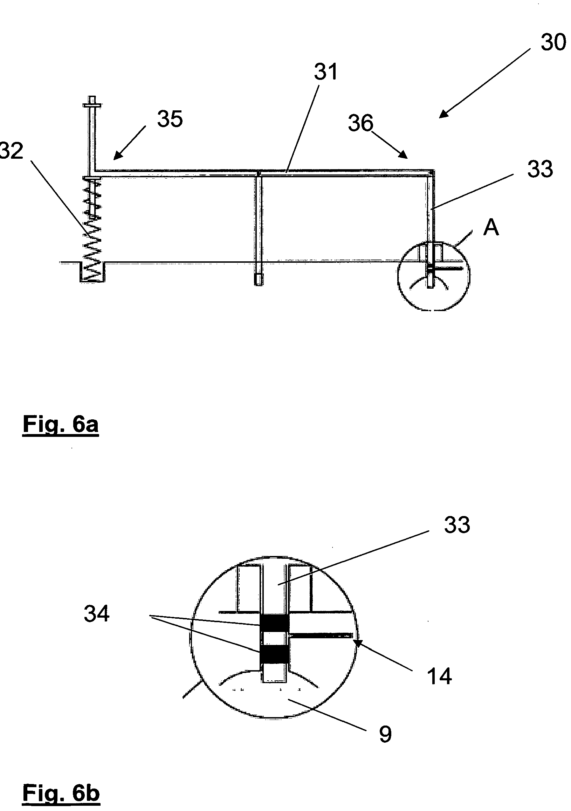

Figur 6a zeigt eine schematische Darstellung eines zweiten Beispiels für ein mechanisches Ventil 30 zum Verschliessen einer Entgasungsöffnung 14 in geschlossener Position. Fig. 6b zeigt eine Detailansicht A aus Figur 6a.

Das Ventil 30 umfasst ein Kipphebel 31, der auf einer Seite 35 mittels einer Feder 32 gestützt ist. Auf der anderen Seite 36 des Kipphebels 31 ist gelenkig eine Dichtstange 33 angebracht, auf der O-Ringe 34 angeordnet sind.

Im geschlossenen Zustand, z.B. wenn die Vorrichtung 1 nicht mit der Basisstation 21 verbunden ist, drückt die Feder 32 die eine Seite 35 des Kipphebels 31 nach oben und die Dichtstange 33 in Richtung des Vorratstanks 9. Wie in Figur 6b ersichtlich ist, sind die O-Ringe 24 dann in einer Position, in welcher die Fluidverbindung zwischen dem Vorratstank 9 und der Entgasungsöffnung 14 unterbrochen ist. Weder Gas noch Flüssigkeit können entweichen.

Wird die eine Seite 35 des Kipphebels 31 gegen die Kraft der Feder 32 gedrückt, was nicht explizit in den Abbildungen dargestellt ist, so wird die andere Seite 36 des Kipphebels 31 und damit die Dichtstange 33 von dem Vorratstank 9 weg gezogen. Die O-Ringe 33 nehmen dann eine Position ein, welcher den Austritt von Gas aus Vorratstank 9 und durch die Entgasungsöffnung 14 erlaubt.

Analog kann eine Vielzahl von Entgasungsöffnungen verschlossen werden.

## Patentansprüche

1. Vorrichtung zur Abgabe von Stosswellen,
- mit einem Behandlungskopf (2), in welchem eine Stosswellenerzeugungsvorrichtung angeordnet ist und der ein Flüssigkeitsreservoir (40) umfasst, wobei die Stosswellenerzeugungsvorrichtung eine elektrohydraulische Stosswellenerzeugungsvorrichtung ist und Elektroden (38) in einem Reflektor (39) angeordnet sind,
- mit einer Steckereinheit (5), die Steckkontakte (7) umfasst zum elektrischen Kontaktieren der Stosswellenerzeugungsvorrichtung mit einer Basisstation (21),
- mit einer Umwälzeinrichtung (8), die mindestens einen vorratstank (9), mindestens eine Pumpe (10) und mindestens eine Entgasungsöffnung (14), bevorzugt ein Entgasungsventil, umfasst, wobei die Umwälzeinrichtung und das Flüssigkeitsreservoir Bestandteile eines abschliessbaren Flüssigkeitskreislaufs sind,
**dadurch gekennzeichnet, dass**
die Umwälzeinrichtung (8) baulich in den Behandlungskopf (2) und/oder die Steckereinheit (5) integriert ist
oder
die Vorrichtung eine Verbindungsleitung (4) zwischen dem Behandlungskopf (2) und der Steckereinheit (5) aufweist, in welcher elektrische Leitungen (6) verlaufen, und die Umwälzeinrichtung (8) baulich in den Behandlungskopf (2), die Steckereinheit (5) und/oder die Verbindungsleitung (4) integriert ist.

2. Vorrichtung gemäss Anspruch 1, **dadurch gekennzeichnet, dass** die Elektroden (38) nachregelbar sind.

3. Vorrichtung gemäss Anspruch 2, **dadurch gekennzeichnet, dass** der Reflektor (39) mit einer Flüssigkeit befüllbar ist, wobei der Reflektor (39) in Richtung der Stosswellenabgabe von einer Koppelmembran (3) abgeschlossen wird.

4. Vorrichtung gemäss einem der vorhergehenden Ansprüche, **dadurch gekennzeichnet, dass** das Flüssigkeitsreservoir (40) des Behandlungskopfes (2) mit einer Flüssigkeit befüllt ist, die nicht lösliches Material, bevorzugt leitende, halbleitenden, polarisierbare Teilchen, Aktivkohleteilchen und/oder Graphitteilchen, enthält.

5. Vorrichtung gemäss einem der vorhergehenden Ansprüche, **dadurch gekennzeichnet, dass** die Entgasungsöffnung (14), insbesondere für den Transport, verschliessbar ist.

6. Vorrichtung gemäss einem der vorhergehenden Ansprüche, **dadurch gekennzeichnet, dass** die Vorrichtung (1) eine Verbindungsleitung (4) zwischen Behandlungskopf (2) und Steckereinheit (5) umfasst, in welcher Fluidleitungen (19) angeordnet sind.

7. Vorrichtung gemäss einem der vorhergehenden Ansprüche, **dadurch gekennzeichnet, dass** die Steckereinheit (5) ein Gehäuse (11), insbesondere ein Kunststoffgehäuse, umfasst, in welchem ein Vorratstank (9), Flüssigkeitsleitungen (12) und/oder eine Aufnahme (13) für eine Pumpe (10) integriert sind.

8. Vorrichtung gemäss einem der vorhergehenden Ansprüche, **dadurch gekennzeichnet, dass** die Steckereinheit (5) elektrische Anschlussmittel und/oder elektronische Steuermittel zur Bedienung der Umwälzeinrichtung (8) umfasst.

9. Vorrichtung gemäss einem der vorhergehenden Ansprüche, **dadurch gekennzeichnet, dass** der Vorratstank (9) eine Flüssigkeitsvolumen zwischen 30ml und 250ml fasst und/oder die Vorrichtung (1) ein Flüssigkeitsvolumen von 80ml bis 350ml fasst.

10. Vorrichtung gemäss einem der vorhergehenden Ansprüche, **dadurch gekennzeichnet, dass** durch die Pumpe (10) eine Leistung von 1.5 bis 10 Watt verrichtbar und/oder einen Durchsatz von 0.5 bis 3.0 Liter pro Minute erzeugbar und/oder einen Druck von 150 bis 1000 mbar aufbringbar ist.

11. Vorrichtung gemäss einem der vorhergehenden Ansprüche, **dadurch gekennzeichnet, dass** der Behandlungskopf (2), die Steckereinheit (5) und die Umwälzeinrichtung (8) oder der Behandlungskopf (2), die Steckereinheit (5), die Umwälzeinrichtung (8) und eine Verbindungsleitung (4) eine gegenüber einer Basisstation (21) auswechselbare Einheit bilden.

12. System zur Erzeugung und Abgabe von Stosswellen mit mindestens einer Vorrichtung (1) gemäss einem der vorhergehenden Ansprüche und einer Basisstation (21), die eine Steckeraufnahme (22) für die Steckereinheit (5) besitzt, wobei die Basisstation einen Hochspannungskondensator aufweist.

13. Verfahren zum Austauschen eines Behandlungskopfes in einem System gemäss Anspruch 12, wobei
der Behandlungskopf (2), die Steckereinheit (5) und die Umwälzeinrichtung (8)
oder
der Behandlungskopf (2), die Steckereinheit (5), die Umwälzeinrichtung (8) und eine Verbindungsleitung (4) als bauliche Einheit von einer Basisstation (21) entfernt und/oder an eine Basisstation (21) montiert werden.

14. Verfahren zum Herstellen einer Vorrichtung zur Abgabe von Stosswellen gemäss einem der Ansprüche 1-11, **dadurch gekennzeichnet, dass** ein Grundkörper (20) für eine Steckereinheit (5), der eine Aufnahme (15) für elektrische Steckkontakte (7) umfasst und insbesondere einen Vorratstank (9), eine Aufnahme (15) für eine Pumpe (10), eine Aufnahme für elektrische Leitungen (6) und/oder Flüssigkeitsleitungen (12) umfasst, im 3D-Druck Verfahren hergestellt wird.

15. Grundkörper für eine Steckereinheit für eine Vorrichtung zur Abgabe von Stosswellen mit einem Behandlungskopf (2), in welchem eine Stosswellenerzeugungsvorrichtung angeordnet ist, insbesondere gemäss den Ansprüchen 1-11, mit einer Aufnahme für Steckkontakte (7) zum elektrischen Kontaktieren der Stosswellenerzeugungsvorrichtung mit einer Basisstation (21), **dadurch gekennzeichnet, dass** in dem. Grundkörper (20) ein Vorratstank (9) und insbesondere eine Aufnahme (13) für eine Pumpe (10) integriert sind, und der Grundkörper aus Kunststoff im 3D-Druckverfahren hergestellt ist.

## Claims

1. Device for delivering shock waves,
- having a treatment head (2) in which a shock wave generating device is arranged and which comprises a liquid reservoir (40), wherein the shock wave generating device is an electrohydraulic shock wave generating device and electrodes (38) are arranged in a reflector (39),
- having a plug unit (5), the plug contacts (7) comprise for electrically contacting the shock wave generating device with a base station (21),
- with a circulating device (8) which comprises
at least one storage tank (9), at least one pump (10) and at least one degassing opening (14), preferably a degassing valve, wherein the circulating device and the liquid reservoir are components of a closable liquid circuit,
**characterized in that**
the circulating device (8) is structurally integrated into the treatment head (2) and/or the plug unit (5)
or
the device has a connecting line (4) between the treatment head (2) and the plug unit (5), in which electrical lines (6) run, and the circulating device (8) is structurally integrated into the treatment head (2), the plug unit (5) and/or the connecting line (4).

2. Device according to claim 1, **characterized in that** the electrodes (38) can be readjusted.

3. Device according to claim 2, **characterized in that** the reflector (39) can be filled with a liquid, the reflector (39) being closed off by a coupling membrane (3) in the direction of the shock wave emission.

4. Device according to one of the preceding claims, **characterized in that** the liquid reservoir (40) of the treatment head (2) is filled with a liquid which contains insoluble material, preferably conductive, semiconductive, polarizable particles, activated carbon particles and/or graphite particles.

5. Device in accordance with one of the preceding claims, **characterized in that** the degassing opening (14) can be closed, in particular for transport.

6. Device according to one of the preceding claims, **characterized in that** the device (1) comprises a connecting line (4) between treatment head (2) and plug unit (5) in which fluid lines (19) are arranged.

7. Device according to one of the preceding claims, **characterized in that** the plug unit (5) comprises a housing (11), in particular a plastic housing, in which a storage tank (9), liquid lines (12) and/or a receptacle (13) for a pump (10) are integrated.

8. Device according to one of the preceding claims, **characterized in that** the plug unit (5) comprises electrical connection means and/or electronic control means for operating the circulating device (8).

9. Device according to one of the preceding claims, **characterized in that** the storage tank (9) holds a liquid volume of between 30ml and 250ml and/or the device (1) holds a liquid volume of between 80ml and 350ml.

10. Device in accordance with one of the preceding claims, **characterized in that** a power of 1.5 to 10 watts can be achieved by the pump (10) and/or a throughput of 0.5 to 3.0 litres per minute can be generated and/or a pressure of 150 to 1000 mbar can be applied.

11. Device according to one of the preceding claims, **characterized in that** the treatment head (2), the plug unit (5) and the circulating device (8) or the treatment head (2), the plug unit (5), the circulating device (8) and a connecting line (4) form a unit which can be exchanged with respect to a base station (21).

12. A system for generating and delivering shock waves comprising at least one device (1) according to one of the preceding claims and a base station (21) having a connector receptacle (22) for the connector unit (5), the base station comprising a high voltage capacitor.

13. A method for replacing a treatment head in a system according to claim 12, wherein
the treatment head (2), the plug unit (5) and the circulation device (8)
or
the treatment head (2), the plug unit (5), the circulating device (8) and a connecting line (4) are removed as a structural unit from a base station (21) and/or mounted on a base station (21).

14. A Method for producing a device for emitting shock waves in accordance with one of claims 1-11, **characterized in that** a basic body (20) for a plug unit (5), which comprises a receptacle (15) for electrical plug contacts (7) and in particular comprises a storage tank (9), a receptacle (15) for a pump (10), a receptacle for electrical lines (6) and/or liquid lines (12), is produced by the 3D printing method.

15. Basic body for a plug unit for a device for delivering shock waves, having a treatment head (2) in which a shock wave generating device is arranged, in particular in accordance with claims 1-11, having a receptacle for plug contacts (7) for electrically contacting the shock wave generating device with a base station (21), **characterized in that** a storage tank (9) and in particular a receptacle (13) for a pump (10) are integrated in the basic body (20), and the basic body is produced from plastic by the 3D printing process.

## Revendications

1. Dispositif pour délivrer des ondes de choc,
- comportant une tête de traitement (2) dans laquelle est disposé un dispositif générateur d'ondes de choc et qui comprend un réservoir de liquide (40), le dispositif générateur d'ondes de choc étant un dispositif générateur d'ondes de choc électrohydraulique et des électrodes (38) étant disposées dans un réflecteur (39),
- comportant une unité à fiche (5), les contacts enfichables (7) comprennent pour mettre électriquement en contact le dispositif générateur d'ondes de choc avec une station de base (21),
- avec un dispositif de circulation (8) qui comprend au moins un réservoir de stockage (9), au moins une pompe (10) et au moins une ouverture de dégazage (14), de préférence une vanne de dégazage, le dispositif de circulation et le réservoir de liquide étant des composants d'un circuit liquide pouvant être fermé,
**caractérisé en ce que**
le dispositif de circulation (8) est intégré structurellement dans la tête de traitement (2) et/ou l'unité de bouchage (5)
ou
le dispositif a une ligne de connexion (4) entre la tête de traitement (2) et l'unité de bouchage (5), dans laquelle les lignes électriques (6) circulent, et le dispositif de circulation (8) est intégré structurellement dans la tête de traitement (2), l'unité de bouchage (5) et/ou la ligne de connexion (4).

2. Dispositif selon la revendication 1, **caractérisé en ce que** les électrodes (38) peuvent être réajustées.

3. Dispositif selon la revendication 2, **caractérisé en ce que** le réflecteur (39) peut être rempli d'un liquide, le réflecteur (39) étant fermé par une membrane de couplage (3) dans la direction de l'émission des ondes de choc.

4. Dispositif selon l'une des revendications précédentes, **caractérisé en ce que** le réservoir de liquide (40) de la tête de traitement (2) est rempli d'un liquide qui contient un matériau insoluble, de préférence des particules conductrices, semi-conductrices, polarisables, de charbon actif et/ou de graphite.

5. Dispositif selon l'une des revendications précédentes, **caractérisé en ce que** l'ouverture de dégazage (14) peut être fermée, en particulier pour le transport.

6. Dispositif selon l'une des revendications précédentes, **caractérisé en ce que** le dispositif (1) comprend une conduite de liaison (4) entre la tête de traitement (2) et l'unité de bouchage (5) dans laquelle des conduites de fluide (19) sont disposées.

7. Dispositif selon l'une des revendications précédentes, **caractérisé en ce que** l'unité enfichable (5) comprend un boîtier (11), en particulier un boîtier en matière plastique, dans lequel sont intégrés un réservoir de stockage (9), des conduites de liquide (12) et/ou un logement (13) pour une pompe (10).

8. Dispositif selon l'une des revendications précédentes, **caractérisé en ce que** l'unité enfichable (5) comprend des moyens de connexion électrique et/ou des moyens de commande électroniques pour faire fonctionner le dispositif de circulation (8).

9. Dispositif selon l'une des revendications précédentes, **caractérisé en ce que** le réservoir de stockage (9) contient un volume liquide compris entre 30 ml et 250 ml et/ou le dispositif (1) contient un volume liquide compris entre 80 ml et 350 ml.

10. Dispositif selon l'une des revendications précédentes, **caractérisé en ce qu'**une puissance de 1,5 à 10 watts peut être atteinte par la pompe (10) et/ou un débit de 0,5 à 3,0 litres par minute peut être généré et/ou une pression de 150 à 1000 mbar peut être appliquée.

11. Dispositif selon l'une des revendications précédentes, **caractérisé en ce que** la tête de traitement (2), l'unité de bouchage (5) et le dispositif de circulation (8) ou la tête de traitement (2), l'unité de bouchage (5), le dispositif de circulation (8) et une conduite de liaison (4) forment une unité qui peut être remplacée par rapport à une base (21) .

12. Système pour générer et délivrer des ondes de choc comprenant au moins un dispositif (1) selon l'une des revendications précédentes et une station de base (21) ayant un réceptacle de connecteur (22) pour l'unité de connecteur (5), la station de base comprenant un condensateur haute tension.

13. Un procédé pour remplacer une tête de traitement dans un système selon la revendication 12, dans lequel
la tête de traitement (2), l'unité enfichable (5) et le dispositif de circulation (8)
ou
la tête de traitement (2), l'unité enfichable (5), le dispositif de circulation (8) et une conduite de raccordement (4) sont retirés d'une station de base (21) et/ou montés sur une station de base (21) comme unité structurelle.

14. Un Procédé de fabrication d'un dispositif d'émission d'ondes de choc selon l'une des revendications 1-11, **caractérisé en ce qu'**un corps de base (20) pour une unité de fiche (5), qui comprend un logement (15) pour des contacts électriques de fiche (7) et en particulier un réservoir de stockage (9), un logement (15) pour une pompe (10), un logement pour lignes électriques (6) et/ou lignes liquides (12), est fabriqué par le procédé d'impression 3D.

15. Corps de base pour une unité enfichable pour un dispositif destiné à délivrer des ondes de choc, comportant une tête de traitement (2) dans laquelle est disposé un dispositif générateur d'ondes de choc, en particulier selon les revendications 1-11, comportant un logement pour des contacts enfichables (7) destinés à venir en contact électrique avec une station de base (21), **caractérisé en ce qu'**un réservoir (9) et en particulier un logement (13) pour une pompe (10) sont intégrés dans le corps de base (20) et le corps de base est fabriqué par impression 3D en plastique.
